# EUROPEAN PATENT APPLICATION

(11) **EP 2 360 222 A2**
(43) Date of publication of application: **24.08.2011**
(21) Application number: 11154732.9
(22) Date of filing: 16.02.2011
(51) Int. Cl.: C09K 3/00

(54) **A substance to be applied in formulations which include at least a first compound and at least a second compound**

(30) Priority: 17.02.2010 AR P000100452
(71) Applicant: Warcok, Maximiliano, CP 1419 Buenos Aires (AR)
(72) Inventor: Warcok, Maximiliano, CP 1419 Buenos Aires (AR)
(74) Representative: Robba, Pierpaolo

(57) **Abstract**

A substance to be applied in formulations which include at least a first compound and at least a second compound, among others, in different proportions, where such application of the compound manages to improve the stability and to increase the viscosity in that formulation. It also refers to the interaction of silicas with liquid substances of an organic type, and to the interaction of the two substances mentioned before with small quantities of an electrolyte. The addition of the latter to a mixture of such silica and such liquid substances produces the flocculation of the disperse silica with the consequent increase of the viscosity, the gelification of the medium, and the modification of other physicochemical properties. Similar viscosities could be obtained with the addition of a higher quantity of silica, with the incorporation of another thickener or another gelifying agent, but in this way the products become more expensive and undesired physicochemical properties are obtained.

## Description

The current invention refers to A substance to be applied in formulations which include at least a first compound and at least a second compound, among others, in different proportions, where such application of the compound manages to improve the stability and increase of viscosity in that formulation. It is also related to the pyrogenic silica, as well as the interaction of this silica with organic liquid substances, and to the interaction of the two substances mentioned above with small quantities of at least one electrolyte.

Commercial products "aerosil^{®} or "cabosil^{®}" are amorphous shapes of silicon oxide, also called "pyrogenic silicon oxides".

These oxides are used, among other applications, as rheological control agents, as dry vehicles for liquids, as emulsifying agents, etc. When they are found in formulations as rheological modifiers, they act on the viscosity of the liquids or semi-liquids. This allows, among other effects, to increase the viscosity, the thickness of the formulation, the formation of gels, paste and it also provides the liquid or semi-liquid with thixotropy.

Thixotropy is the property that a liquid or semi-liquid has of reducing its viscosity while a power is applied, such as pressure or agitation. When the application of that power is stopped, and after a while, the liquid or semi-liquid increases its viscosity again to one close to the initial viscosity.

At present, there are a great number of formulations that use pyrogenic oxides in combination with other substances. In many of these formulations it is common the use of other organic thickeners of the polyacrylate kind (carbopoles®), carboxymethylcellulose, hydroxymethylcellulose, among others. The function of these thickeners is to increase the viscosity even more, without the need for adding great quantities of silica. This combination of thickeners and silica is performed in order to avoid inconveniences such as
- The increase in the cost of the product.
- The reduction in the drying time, in some formulations where this is unfavourable (this effect is due to the larger contact surface of the solvent with the silica particles).
- The obstruction of ducts where the compound passes (this is because of the great quantity of suspension solids)
- The productive difficulty of preparing compounds with a high percentage of silica because very large containers would be necessary to produce little product given the very low density of pyrogenic silica and, in addition, for silica to get mixed with the liquid substances, time and very slow agitation are needed to avoid losing silica through the air stream generated by the agitation.

Besides, several inconveniences can be found according to the application and the formulation. Other inconveniences arise when using the organic gelling agents mentioned before, for example, in formulations of gels to maintain the fondue hot. In these cases silica, ethanol and additional gelling agents such as polyacrylates, hydroxymethylcelluloses, polyacrylamides or any other product which contributes to the thickening of the formulation are used. These gelling agents or thickeners which are additional to the silicon oxides generate carbon residues that are deposited on the pot of the fondue and/or generate some small explosions during the operation with the consequent projections of hot material.

With the method described here these inconveniences are solved, keeping the advantages of the already mentioned gelling agents, omitting the incorporation of the additional gelling agents and replacing them with at least one electrolyte. These aggregates are solid or dissolved in solution in strengths varying from 0.001% mass in mass to the solubility limit allowed by solvents.

It is well known that in formulations of gels for sliding inhibitors to use on the skin, in the feet, hands and elements such as Addherence^{®}, a product consisting in ethanol or isopropanol, the aforementioned additional silicas and gelling agents. Addherence^{®} in the form of gel is unstable, few days after being prepared it exudes alcohol, losing its homogeneity. With the replacement of these gelling agents with the mentioned at least one electrolyte, in strengths lower to the ones of the gelling agents, a homogeneity of 2 years is achieved, proved by an accelerated aging study. It also improves the drying speed, the final adherence that generates on the skin or on the element where it is applied, admitting a formulation with much lower aqueous content, which generates a lower drying time of the product, in addition to helping to dry the treated surface in case it was already wet.

Therefore, an object of the current invention is the provision of a substance to be applied in formulations which include at least a first compound and at least a second compound, among others, in different proportions, where such application of the compound manages to improve the stability and to increase the viscosity in that formulation, where such substance is at least an inorganic electrolyte.

The current invention refers to the interaction of these silicas with liquid substances of the organic kind, such as the ones described below, and to the interaction of the two substances with small quantities of at least one electrolyte, as the ones described below. The incorporation of a percentage (between 0.001% and 10%) of the latter to a mixture comprised of at least a silica and at least an organic liquid substance, produces the flocculation of the disperse silicas with the consequent increase of viscosity, the gelification of the medium and the modification of other physicochemical properties which are maintained with the passing of time and are stable. Similar viscosities could be obtained with the addition of a higher quantity of silica, with the incorporation of another thickener or another gelifying agent, but in this way the products would become more expensive and physicochemical properties that could be undesired for the application intended for these mixtures could be obtained, as it has been mentioned before.

The liquid substances of the organic type used are primary, secondary or tertiary short chain alcohols and polyalcohols up to 10 carbon atoms, branched or not branched, such as Methanol, Ethanol, isopropanol, butanol, terbutanol, propanol, isobutanol, glycerin, ethylene glycol, propylene glycol, polyethylene glycols of different molecular masses, propylene glycols different molecular masses, etc.

The electrolytes used are Inorganic, preferably the ones which confer an alkaline medium when dissolved in water. It is at least one from a group consisting of sodium hydroxide, potassium hydroxide, sodium phosphates, potassium phosphates, ammonium phosphates, sodium carbonates, sodium acid carbonate, potassium carbonates and potassium acid carbonate. These are solid or dissolved in solution aggregates in strengths varying from 0.001% mass in mass to the solubility limit admitted by the solvents.

The following tables indicate the increase of dynamic viscosity measured with a Brookfield RVT equipment in the different formulations. Ethanol 96° is used as an example of an organic liquid; sodium acid carbonate at 10% in aqueous solution is used as an example of an electrolyte, and the chosen silica is Aerosil^{®} 200.

| Number of example | % mass in mass of Silica in ethanol 96° | % mass in mass of NaHCO₃ in final mixture | Viscosity in cSt | Observations after 24 hours |
|---|---|---|---|---|
| 1 | 5.00 | 0.00 | 400 | NOT STABLE |
| 2 | 5.00 | 0.01 | 400 | NOT STABLE |
| 3 | 5.00 | 0.05 | 440 | NOT STABLE |
| 4 | 5.00 | 0.10 | 660 | NOT STABLE |
| 5 | 7.50 | 0.00 | 1100 | NOT STABLE |
| 6 | 7.50 | 0.005 | 1200 | STABLE |
| 7 | 7.50 | 0.0075 | 1820 | STABLE |
| 8 | 7.50 | 0.1 | 6000 | STABLE |
| 9 | 10.00 | 0.00 | 6000 | STABLE |
| 10 | 15.00 | 0.00 | 944000 | STABLE |

When comparing example 5 and example 6 we can observe that the addition of 0.005% mass in mass of sodium acid carbonate causes the mixture to increase its viscosity and to get stabilized.

When comparing example 5 and example 7 we can observe that the addition of 0.0075% mass in mass of sodium acid carbonate causes the mixture to increase its viscosity by approximately 65%.

When comparing example 5 and example 8 we can observe that the addition of 0.1% mass in mass of sodium acid carbonate causes the mixture to increase its viscosity by approximately 445%.

When comparing example 8 and example 9 we can observe that the addition of 0.1% mass in mass of sodium acid carbonate causes the mixture to increase its viscosity 6000 cSt (centiStoke). Identical viscosity is achieved with the addition of 10% of silica. This implies a relative saving of 25% of silica, reaching, thus, the solution to several economic and technological problems.

## Claims

1. A substance to be applied in formulations which include at least a first compound and at least a second compound, among others, in different proportions, where such application of the compound manages to improve the stability and to increase the viscosity in that formulation, characterized because such substance is at least one inorganic electrolyte.

2. The substance in accordance with claim 1, characterized because such at least one electrolyte is applied to such formulation in a proportion of 0.001% to 10% in weight.

3. The substance in accordance with claim 1, characterized because such at least one electrolyte is diluted in a solution in a proportion between 0.1% and 10% of mass in mass of solvent.

4. The substance in accordance with claim 1, characterized because such formulation presents other compounds in addition to the aforementioned first and second compounds.

5. The substance in accordance with claim 4, characterized because such other compounds are present in a proportion lower than the proportion in which such at least one first compound is found.

6. The substance in accordance with any of the preceding claims, characterized because such first compound comprises at least one primary, secondary or tertiary short chain alcohol, polyalcohol up to 10 carbon atoms, branched or not branched.

7. The substance in accordance with any of the preceding claims, characterized because such second compound is comprised of at least one pyrogenic silica.

8. The substance in accordance with claim 1, characterized because such at least one inorganic electrolyte is of the kind which confers an alkaline medium when dissolved in water.

9. The substance in accordance with claim 8, characterized because such inorganic electrolyte is at least one of a group consisting of sodium hydroxide, potassium hydroxide, sodium phosphates, potassium phosphates, ammonium phosphates, sodium carbonates, sodium acid carbonate, potassium carbonates and potassium acid carbonate.
